# EUROPEAN PATENT APPLICATION

(11) **EP 1 270 533 A2**
(43) Date of publication of application: **02.01.2003**
(21) Application number: 01129367.7
(22) Date of filing: 17.12.2001
(51) Int. Cl.: C04B 38/06, C12N 5/00, A61L 27/10

(54) **Ceramic materials, method for their production and use thereof**

(30) Priority: 21.06.2001 GB 0115204
(71) Applicant: Zellwerk GmbH, 16727 Eichstädt (DE)
(72) Inventor: Hoffmeister, Hans, H.W., 16727 Eichstädt (DE)
(74) Representative: HOFFMANN - EITLE

(57) **Abstract**

The present invention relates to ceramic materials comprising a crystalline core oxide ceramic dispersed with an amorphous polyanionic intergranular phase as well as methods and materials for the production of said ceramic materials, articles comprising said ceramic materials and uses thereof.

## Description

The present invention relates to ceramic materials comprising a crystalline core oxide ceramic dispersed with an amorphous polyanionic intergranular phase as well as methods and materials for the production of said ceramic materials, articles comprising said ceramic materials and uses thereof.

### Background of the Invention

The culture of cells in fermentors and bioreactors is currently used for the production of a variety of organic substances that are either not obtainable or difficult or expensive to obtain in other ways. Suitable cells, for example, selected cell clones or cell clones altered by genetic engineering, are capable of synthesizing small organic molecules such as vitamins, lipids, nucleotides, hormones, amino acids as well as more complex molecules such as nucleic acids, polypeptides including enzymes and other proteins and even complex glycoproteins.

A number of important biopharmaceuticals can be produced in cell culture. For example, various forms of erythropoietin, Factor VIII, Factor IX, Factor VIIa, tissue plasminogen activator (tPA), thyrotropin-alpha, follicle stimulating hormone (FSH), interferon-beta 1a, dornase-alpha, and several antibodies and antibody fragments are currently produced in mammalian cells and have been approved as pharmaceuticals in the US or European Union. Moreover, PDGF (platelet-derived growth factor), hirudin and hepatitis B surface antigen (HbsAG) are currently produced in yeast and E. coli is used to produce modified tPA, insulin, human growth hormone, GM-CSF, interferon-alpha 2a, interferon-alpha 2b, interferon-beta 1b, interleukin-11, beta-glucocerebrosidase, and TNF-alpha (tumor necrosis factor-alpha).

However, methods for the production of such molecules are often complex and costly because, for example, high cell densities of suitable anchorage-dependent cells are difficult to achieve in bioreactors and other culture facilities and the supports used in bioreactors and other culture facilities for cell growth may not be reusable.

Different supports have been used in bioreactors for increasing the growth surface in order to achieve a greater cell density, i.e. more cells per volume, in culture vessels. A number of organic polymer matrices are available and have been mainly recommended for this purpose (for example, porous dextrans, celluloses, nylon fabrics, and collagen (1-8). In addition, porous glasses (for example, porous glass ceramics such as Siran (7-9), solid pieces of alumina (10) and alumina honey combs (11) have been used.

High cell densities can be achieved with organic matrices in bioreactors at the laboratory scale, but are not suitable for large volume fermentors and bioreactors that are required for cell culture at the industrial level. Their structures are deformed and their pores are pressed together in fixed-bed reactors. The materials are also expensive because they can only be used once.

The porous glasses do not have these disadvantages and only certain glass compositions may be suitable (7). However, the growth of many cell types on these surfaces is slow.

Ceramic materials derived from bone substances (apatite, tricalcium phosphate compounds) that have been employed as biodegradable, biocompatible and bioactive implants have also been described (12-14, 30-32). Bone-forming cells (osteocytes, osteoblasts), as well as other anchorage-dependent cells such as fibroblasts have been cultured in vitro and in vivo on these ceramics. The influence of structure, crystal structure, surface form, polymeric organic additives, mixtures with oxide ceramic materials, sintering regimes and other factors on growth, adhesion and formation of tissue structure of the cells has been tested along with the process of mineralization in tissue (15-21, 33-34).

Further oxide ceramics composed of different elements, among them aluminum oxide, titanium oxide and zirconium oxide in the form of glasses, crystalline structures and composites with organic polymers, have been examined as to their suitability as biocompatible, bioinert materials, as bone substitutes and for fillings for teeth (35-42)

Some products of oxide ceramics are produced as honeycombed bodies, macroporous particles and formed parts. They serve, among others, for the dense colonization with microorganisms for water purification in sewage treatment plants (10).

However, a support which enables the seeding, growth, division, reproduction, differentiation, continuance of a differentiated state, maintenance and/or culture of anchorage-dependent eukaryotic cells and prokaryotic cells that is resistant to the physical and chemical strains of such systems and can be repeatedly reused is greatly needed in order to reduce the complexities and expense of large-scale fermentation.

Accordingly, an object of the present invention is to make ceramic materials available that provide an improved support for the culture of anchorage-dependent eukaryotic cells and prokaryotic cells that can be repeatedly sterilized and maintain its structural and chemical integrity.

A further object of the present invention is to provide an article of manufacture that comprises said material and permits the improved culture of anchorage-dependent eukaryotic cells and prokaryotic cells and/or improved production of organic molecules, particularly polypeptides and proteins, in culture from anchorage-dependent eukaryotic cells and prokaryotic cells.

A further object of the present invention is to provide a method for the production of organic compounds, particularly polypeptides, proteins, glycoproteins using said material.

Other objects will be apparent from the specification in light of the background art.

### Summary of the Invention

The present invention provides a ceramic material comprising a crystalline core oxide ceramic dispersed with an amorphous polyanionic intergranular phase.

In addition, the present invention provides an article for culturing anchorage-dependent eukaryotic cells or prokaryotic cells comprising a crystalline core oxide ceramic dispersed with an amorphous polyanionic intergranular phase.

Moreover, the present invention provides a method for the production of a ceramic material comprising ceramic materials comprising a crystalline core oxide ceramic dispersed with an amorphous polyanionic intergranular phase.

Furthermore, the present invention provides a slurry, pressing powder or plastic ceramic material for use in the different methods for the production of said ceramic material and articles made therefrom as well as the green bodies comprising said starting materials.

The present invention also provides a method for culturing anchorage-dependent eukaryotic cells or prokaryotic cells using a ceramic material or article comprising a crystalline core oxide ceramic dispersed with an amorphous polyanionic intergranular phase.

In addition, the present invention provides a method for the production of organic compounds comprising culturing anchorage-dependent eukaryotic cells or prokaryotic cells on a ceramic material or article comprising a crystalline core oxide ceramic dispersed with an amorphous polyanionic intergranular phase, in the presence of a culture medium and isolating said organic compound from said cell or culture medium.

### Brief Description of the Drawings

Figure 1: A light microscopic picture showing the porous, open-celled nature of a multiphase ceramic material surface (zirconia/phosphate) used for culturing cells.

Figure 2: A light microscopic picture with HeLA cells grown on a dense burned disc with micro-roughness by dragging. The material is zirconia with amorphous phosphate phase. After stopping cell growth cells were stained with Giemsa solution.

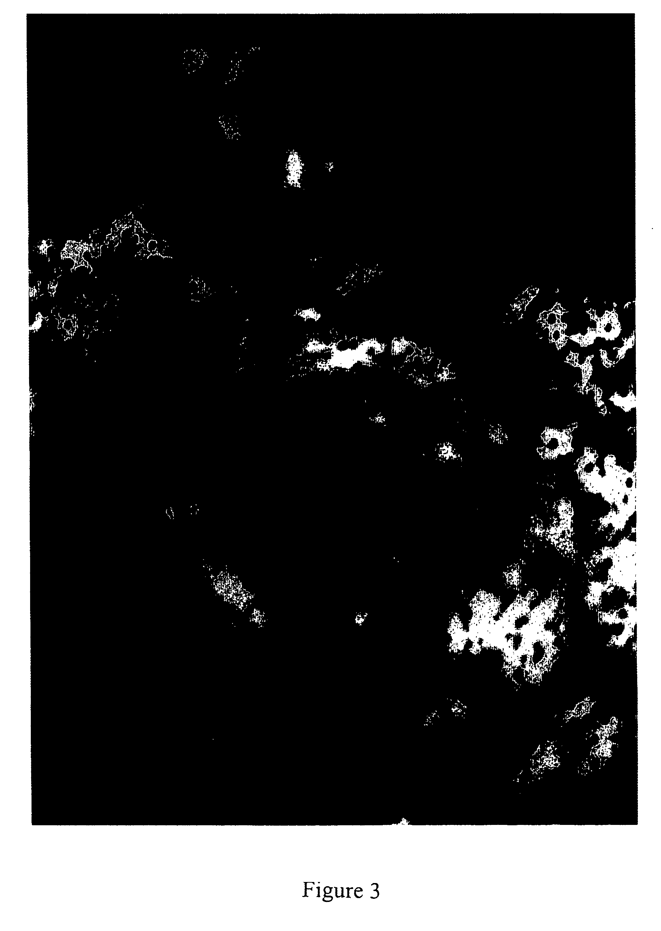

Fig. 3: A light microscopic picture of CHO cells grown on macro-porous particles from multiphase ceramic material(zirconia/phosphate, Giemsa staining).

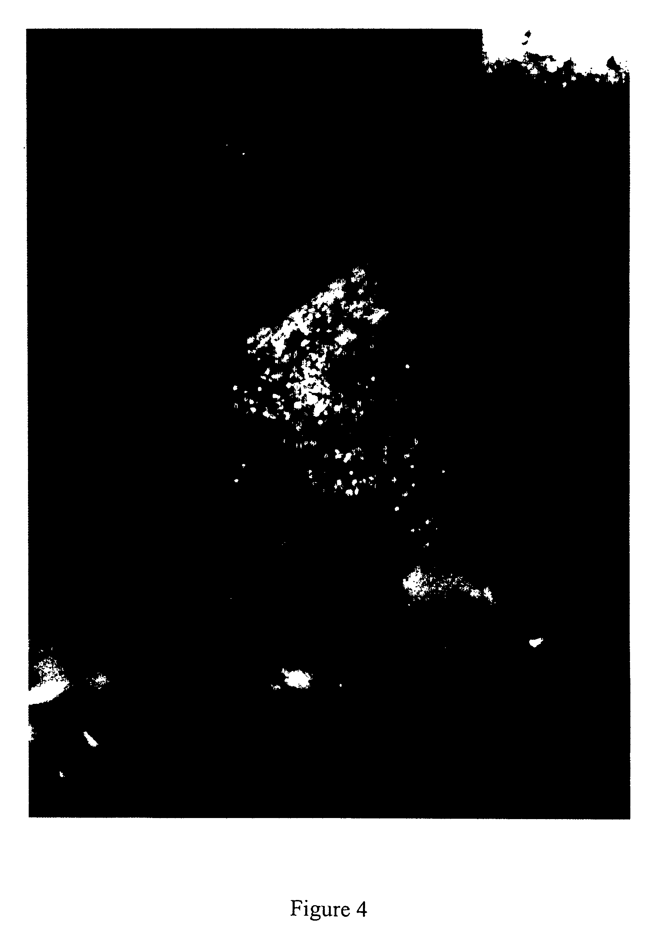

Fig 4: Macro-porous particles from alumina/silicon core ceramic with amorphous phosphate phase. Co-cultured human hepatocytes and mouse fibroblasts (Hep G2, L 929, Giemsa stain).

Figure 5: Data curves showing the improved growth of CHO-K1 cells on porous zirconia/phosphate particles compared to porous glass particles (Siran).

Figure 6a/6b: X-ray diagram of a typical multiphasic ceramic material (zirconia, fully stabilized with calcium, 2 % phosphate added) showing characteristic peaks of crystalline and amorphous phases. The sharp peaks 3-7 are derive from cubic zirconia. The broad bands 1 and 2 show amorphous polyanionic phosphate phases, as can be seen from Fig. 5b. This figure shows the magnified left part of Fig.5a. The lower line in this figure is cubic zirconia without phosphate.

### Detailed Description of the Invention

As a result of their physical and chemical properties, ceramic materials comprising oxides, such as zirconium oxide and aluminum oxide, are used for the manufacture of a wide variety of articles such as ceramic valves, plungers, pistons, milling beads, parts subject to wear.

The addition of small amounts of polyanionic compounds to a slip comprising various oxide ceramics can lead to ceramic materials that are comprised of crystalline oxide ceramics on the one hand and amorphous polyanionic intergranular phases on the other hand.

As a result of their chemical composition, their surface properties and, in particular uses, their porosity, roughness and form, it has been surprisingly found that oxide ceramics that additionally contain various amounts of an amorphous polyanionic intergranular phase, are exceptionally well suited for culturing anchorage-dependent eukaryotic cells and prokaryotic cells.

Thus, the present invention provides a ceramic material comprising a crystalline core oxide ceramic dispersed with an amorphous polyanionic intergranular phase.

As such, the ceramic material according to the invention is a multiphase ceramic material comprising at least one crystalline phase and at least one amorphous phase. However, the ceramic material of the invention can also include other phases. For example, the ceramic material of the invention can contain a crystalline polyanionic intergranular phase as well. Preferably, the multiphase ceramic material of the present invention is a triphasic ceramic material or a biphasic ceramic material. A preferred triphasic material is a ceramic material comprising a crystalline core oxide ceramic dispersed with an amorphous polyanionic intergranular phase. Besides the amorphous phase components of the polyanionic material occur as a crystalline intergranular phase depending of the sintering process. The faster the cooling process after sintering the lower the amount of crystalline intergranular phase. Preferably, the amorphous polyanionic phase in the ceramic materials of the invention comprises about 75%, more preferably about 90% of the total polyanionic intergranular phase of the ceramic material.

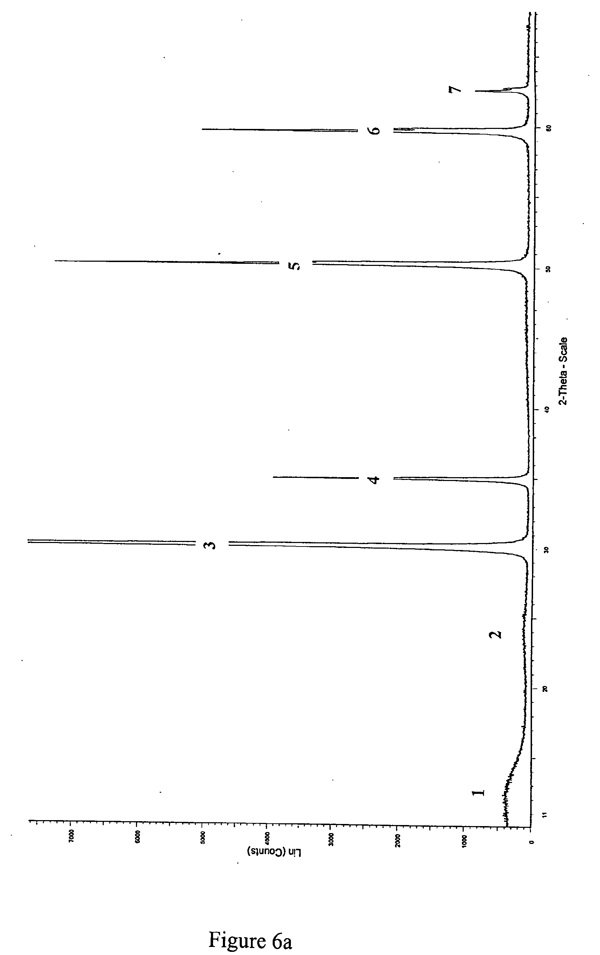

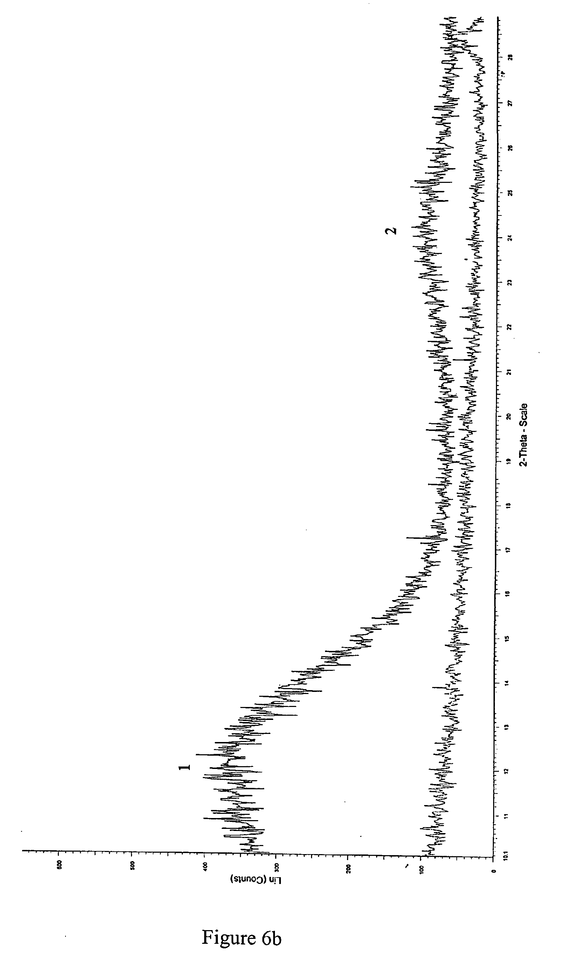

The nature of the multiphasic ceramic can be determined by quantitative X-ray phase analysis (for example using Bruker D 500 X-ray equipment). Fig.6 shows a typical diagram of an oxidic core ceramic (ZrO₂) containing 17% calcium oxide as a stabilizing agent, 4% phosphate in form of an amorphous intergranular phase and around 0.4% of crystalline intergranular phase. The amorphous part of the ceramic can be determined precisely by mixing the fine ground ceramic powder with a known amount of an internal standard (for example silicon) and then performing the X-ray phase analysis.

The crystalline oxide ceramic of the ceramic material of the present invention provides a core material having high physical strength (bending strength is between 200 and 800 Mpa). The multiphasic oxide ceramics according to the invention are resistant to both physical stresses such as strain, temperature and chemical stresses such as the exposure to basic or acidic liquids.

An oxide of the crystalline core oxide ceramic is preferably selected from the group consisting of zirconium (IV) oxide, aluminum oxide, silicon (IV) oxide, titanium (IV) oxide, hafnium (IV) oxide, cerium (IV) oxide and mixtures thereof.

However, any oxide that provides a crystalline core oxide ceramic that is resistant to the above mentioned physical and chemical stresses to the same degree as those provided by the preferred oxide starting materials can be used as a starting oxide material to produce the ceramic material of the present invention.

In a preferred embodiment, the oxide of the crystalline core oxide ceramic comprises zirconium (IV) oxide stabilized with about 1 to about 18 mol% calcium oxide or about 1 about 10 mol% magnesium oxide or about 1 to about 10 mol% yttrium oxide. When Zirconium (IV) oxide is used it is preferably fully stabilized with about 17 mol% CaO.

The starting oxide of the crystalline core oxide ceramic used is preferably present in the ceramic material of the present invention in an amount of about 70 to about 99.5% with respect to the weight of the total ceramic material after sintering, more preferably about 90 to about 99%, most preferably, about 94 to about 98%.

A polyanionic compound in the amorphous intergranular phase is preferably selected from the group consisting of phosphates (for example, metaphosphoric acid and salts thereof, orthophosphoric acid and salts thereof, manganates (for example, manganic acid and dioxomanganates (IV), molybdates (for example, molybdic acid and salts thereof), tungstenates (for example, orthotungstic acid and salts thereof) or mixtures thereof.

The polyanionic compound is preferably present in the ceramic starting material of the present invention in an amount of about 0.5 to about 30% with respect to the weight of the total ceramic material after sintering, more preferably about 1 to about 10%, most preferably about 2 to about 6%.

In addition, as a result of the amorphous nature of the polyanionic intergranular phase, the surface of the ceramic material according to the invention possesses specific characteristics that allow the improved culture of anchorage-dependent eukaryotic cells and prokaryotic cells. Depending on the amount of added polyanionic compound, the hydrophilicity of the surface of the ceramic material according to the invention can be varied and set over a wide range.

For applications of the ceramic material of the present invention involving the culture of eukaryotic cells and prokaryotic cells, the portion of amorphous polyanionic compounds in the ceramic can be selected in such a manner that the surface of the ceramic material possesses an adhesive strength that allows for advantageous cell adhesion with respect to growth, division, reproduction, differentiation or continuance of a differentiated state.

The degree of hydrophilicity of the surface of the ceramic material is mainly responsible for the adhesive strength and can be determined by measuring the contact angle between water and the ceramic surface (for example with a Wet-3000, Rhesca Co Ltd., Tokyo) (23). The contact angle of ceramic surfaces with water can therefore be used as a physical parameter determining the adhesive strength between cells and supports.

Portions of polyanionic compounds between about 0.5% and about 30% result in ceramic materials that have physical and chemical properties that are particularly suitable for in vitro cell culture. Contact angles between about 65° and about 40° can thus be achieved. Preferably, contact angles between about 48 and about 52 are achieved.

However, the preferred amount of polyanionic compounds in the ceramic material according to the invention is between about 1% and about 10%. This leads to contact angles in our multiphasic ceramic materials between about 60° and about 45°. More preferably, the amount of polyanionic compounds in the ceramic material according to the invention is between about 2% and about 6%. In this case, contact angles about 50° result.

On the one hand, anchorage-dependent eukaryotic cells adhere to the ceramic material of the invention with portions of polyanionic compounds between about 0.5% and about 30% sufficiently strong to achieve high cell densities and high viabilities. On the other hand, the adhesiveness is weak enough to ensure light separation during cell division. This property leads to high cell division rates.

The above described multiphasic ceramic materials according to the invention can be produced using convenient methods. The respective core ceramic oxide, (optionally mixed with a stabilizing oxide, is subjected to wet grinding to establish a fine slip. Typically, after the wet grinding process is finished the polyanionic compound is mixed into the slip. This slip can be used to produce slurry for filling moulds, dry powder for uniaxial or isostatic pressing (by drying at 50°C and afterwards dry milling to get pressing powder for example) and plastic ceramic material to form bodies.

Additives like one or more binder(s) and/or liquidizer(s) can be used.

Among the additives that can be used are one or more support materials that are capable of being burned off during sintering. Preferably, said support materials are organic materials. More preferably, said organic material is selected from the group of graphite, polymeric compounds such as polyethylene, polypropylene, polysterol, latex, and coffee grinds. Scaffolds from polyurethane and other organic materials can be soaked with slip, dried and burned out.

Furthermore, the present invention provides a slip, a pressing powder or a plastic material for use in the method for the production of said ceramic material as well as a green body comprising said slip starting material.

The slip or pressing powder or plastic ceramic material according to the invention comprises
a) one or more oxides capable of forming a crystalline oxide ceramic at a sintering temperature of about 1150°C to about 1600°C
b) one or more polyanionic compounds capable of forming an amorphous polyanionic intergranular phase at a sintering temperature of about 1150°C to about 1600°C and, optionally,
c) one or more binder(s), liquidizer(s) and/or support material(s) that volatilize or burns off by a sintering temperature of about 1150°C to about 1600°C.

Oxides, polyanionic compounds optional additives and preferred embodiments thereof are those described above.

In particular, the slip or pressing powder or plastic ceramic material of the present invention can comprise an additive, preferably a support material, according to the invention that is capable of being burned off during sintering such as organic particles, preferably of a defined size range depending on the eventual use of the slip, of graphite, polyethylene etc., or structured forms from polyurethane foam, polystyrol, etc., which result in the formation of bubbles or pores or other hollow spaces upon sintering.

The slip of the present invention can also be "densified" such that, after sintering, a dense block of the ceramic material is obtained that can be cut, crushed, ground or milled into particles, pellets, balls, sheets , (other forms here). Dense ceramic materials according to the invention having a porosity of less then about 1% is obtained by using fine grained oxide starting material (particles of about 200 to about 1000 nm in size).The slurry can be dried at about 50°C and then dry grounded to generate pressing powder. Isostatic or uni-axial pressed and sintered bodies lead to dense material when sintering temperatures from about 1500°C to about 1600°C are used.

The green body of the invention comprises a slip according to the invention and, optionally, a form containing one or more materials that are capable of being burned off during sintering at a temperature about 800°C. Preferably, said materials that are capable of being burned off are organic materials. More preferably, said organic material is selected from the group of polymer plastics such as polyurethane, polyethylene, polypropylene, polysterol, latex.

The shape of the form comprising a material that is capable of being burned off during sintering can vary depending on the intended use of the ceramic material according to the invention. Form of a particle, pellet, sphere, disc, plate, film or monolithic element.

The present invention also provides a method for the production of a ceramic material or article comprising a crystalline core oxide ceramic dispersed with an amorphous polyanionic intergranular phase, comprising the steps of mixing a slip comprising:
a) one or more oxides capable of forming a crystalline oxide ceramic at a temperature of about 1150 to about 1600°C
b) one or more polyanionic compounds capable of forming an amorphous polyanionic intergranular phase at a temperature of about 1150 to about 1600°C
c) one or more binder(s), liquidizer(s) and/or support material(s) that volatilize or burns off by a sintering temperature of about 1150°C to about 1600°C.
and sintering said slip, or a formed green body comprising said slip and a material that is capable of being burned off during sintering at a temperature at a temperature range from about 1150°C to about 1600 °C. When the method is used with a high content of tungstenate of about 25 to 30% for example, the sintering temperature can lie at about 1150°C; with Al₂O₃ and phosphate, for example, about 1600°C. The skilled person will know how to adjust the sintering temperature to the composition of the components.

The surface of the ceramic material according to the invention can be made to have varying degrees of roughness and porosity depending on the intended use.

By selecting certain particle sizes of the oxide ceramic starting materials and suitable sintering regimes, the surface area and porosity of the ceramic material according to the invention can be set over a wide range. Surfaces having a defined microporosity can also be created that have large fissured surfaces.

The porosity of the ceramic material according to the invention can range from about 60% to about 95% (porosity is defined as the percentage volume of pores compared with the total volume of the support).

The surface area of the ceramic material according to the invention can range from about 1000 cm²/ml to about 6000 cm²/ml.

The size of the pores of the ceramic material according to the invention can range from about 10 nanometers to about 10 millimeters. In case of larger pores the pore size will be determined by the size and form of organic particles to be burned off from the green body or by respective foam bubbles generated in the slip. The size of micropores is mainly dependent on the grain size of the starting ceramic powder, the sintering temperature and the sintering regime.

The particle size of the oxide ceramic starting material ranges between about 0.5 µm to about 2 µm. Preferably, particles from about 0.6 µm to about 1 µm are used.

For applications involving the culture of anchorage-dependent eukaryotic cells and prokaryotic cells, it is preferable to have surfaces with a relatively high surface area. In addition, the presence of many, open-celled macropores impart large internal surfaces and ensure permeability with medium. An additional microporosity of the surfaces can be created that increases the area further and creates anchoring points for the cells. The surfaces of the macroporous and the densely sintered ceramics can be additionally enlarged by applying microporous layers of the same material or by creating microroughness. Fissured surfaces with micropores in the range of about 0.5 to about 10 µm are covered by anchorage-dependent eukaryotic cells and prokaryotic cells more quickly and more densely than smooth or polished surfaces, independent of the type of ceramic.

For applications involving the culturing of anchorage-dependent eukaryotic cells and prokaryotic cells the cell supports may comprise two layers. First a macroporous skeleton/core of multiphase oxide ceramic is densely sintered using a slip with particle sizes from about 0.6 µm to about 1 µm. The resulting support is then soaked with a thin slip layer consisting of the same multiphase oxide ceramic but having preferred particle sizes that are different as those used for the first layer and range from about 1 µm to about 10 µm, more preferred from about 2 µm to about 4 µm. To form a microporous, fissured surface, this second layer is sintered at about 900°C to about 1400°C. Thus, in a further embodiment of the invention, the surface area of the ceramic material of the invention is preferably from about 5000 cm²/ml to about 6000 cm²/ml.

In a still further embodiment, the porosity of the ceramic material of the invention is preferably from about 75% to about 90%.

Such open-pored ceramic material according to the invention is suitable for industrial cell culture on a large-scale. The ceramics can be reused many times and any means of sterilization can be used.

The ceramics according to the invention are especially suited as a support for the culture of anchorage-dependent eukaryotic cells and prokaryotic cells.

The term "culture" or "culturing" used herein is meant to include the seeding, growth, division, reproduction, differentiation or continuance of a differentiated state or maintenance of anchorage-dependent eukaryotic cells and prokaryotic cells in vitro under conditions that are appropriate for a particular cell type. The skilled person will be familiar with establishing the growth medium and atmospheric requirements of a particular cell.

The cells are supported by the ceramic material according to the invention, replicate thereon and remain viable there for long periods when they are cultured in customary nutrient medium with the aid of the ceramic materials of the invention. The degree of differentiation of primary anchorage-dependent eukaryotic cells is maintained over longer periods of time than when culturing such cells on customary supports (for example, glass, plastic). A pretreatment of the surface of the ceramic material according to the invention (for example with fibrin etc.) is also not required after multiple uses of the ceramic formed parts.

The ceramic material according to the invention and articles manufactured therefrom can be used as a support for the culture of any anchorage-dependent eukaryotic cell such as animal cells, including human cells, insect cells, plant cells, yeast cells, or prokaryotic cells.

The term "anchorage-dependent eukaryotic cell" is used herein to signify one or more eukaryotic cells, cell types, cell lines, or subclones or mixtures or derivatives thereof that require a surface for seeding, growth, division, reproduction, or continuance of a differentiated state or maintenance in culture or are capable of seeding, growth, division, reproduction, differentiation or continuance of a differentiated state or maintenance in culture on a surface. Such cells include mortal or immortal primary cells prepared directly from the tissues or organs of a donor and cells that have been transformed to an immortal phenotype via spontaneous chemical or physical mutagenesis event(s).

Examples of anchorage-dependent eukaryotic animal cells in accordance with the present invention include mammal cells. These cells can optionally be altered by genetic engineering and used for producing recombinant peptides, proteins and glycoproteins. Preferred anchorage-dependent eukaryotic mammalian cells in accordance with the present invention are human cells, rodent cells such as CHO cells, BHK cells, HEK cells, COS cells; primate cells including L cells, endothelial cells, 3T3 cells, tumor cell lines such as HeLa cells, hepatocytes.

In a preferred embodiment, the anchorage-dependent eukaryotic cells are naturally occurring or genetically altered human cells or cells derived therefrom. Such cells are suitable for the production of autologous and homologous cells for therapeutic purposes arid other clinical uses. Anchorage-dependent mammalian cells that are obtained for example from blood and organs of living donors or from surgical preparations from patients are also considered as useful for the present invention.

Cells from further animal species can be advantageously cultured as well on the multiphasic ceramic supports according to the invention. For example, avian cells such as CEF, MDCC/MBS1, NA101, PA1-CEF, ECACC, IZSBS cells; fish cells such as BB,BF2, RTH, FHM, D11, A2, RTG2 cells; reptilian cells such as FT bullfrog cells, can be employed.

Preferable insect cells include Drosophila melanogaster cells, SF9, SF21, SPC158, MB-L2, BM5 cells.

Preferable yeast cells include such as Saccharomyces, for example, S. cerevisiae, S. carlsbergensis, Pichia pastoris, Kluyveromyces lactis, Hansenula polymorpha, Aspergillus niger, Nerospora crassa.

Preferable prokaryotic cells include gram-positive such as Bacillus subtilis, B. cereus, B. thuringiensis, B.brevis and B. megaterium and gram-negative bacterial cells such as E. coli.

Anchorage-dependent eukaryotic cells and prokaryotic cells in accordance with the invention can be eukaryotic cells or prokaryotic cells that occur in nature or that have been or are altered to produced one or more organic compounds, preferably polypeptide(s) and proteins, at a level higher than that produced by said cell in nature.

Anchorage-dependent eukaryotic cells and prokaryotic cells can be altered by genetic engineering techniques well known in the art, including the stable or transient introduction of one or more exogenous nucleic acids by transfection, transformation, viral or phage infection, etc. Such cells will either express one or more proteins that indirectly lead to the production of organic molecules, for example, one or more enzymes in a biosynthetic or biodegrative pathway for the production of, for example, vitamins, lipids, nucleotides, hormones, amino acids, etc. or directly express one or more exogenous polypeptide(s) and proteins of interest.

Anchorage-dependent eukaryotic cells and prokaryotic cells may also be altered by introducing nucleic acids that encode polypeptides and proteins which allow for the selection of cells containing said nucleic acid(s), for example, by antibiotic resistance or growth requirements in a particular medium or optical determination such as the presence of the GFP (green florescent protein).

The invention provides a method for the production of anchorage-dependent eukaryotic cells or prokaryotic cells comprising contacting the ceramic material or article according to the invention with eukaryotic cells or prokaryotic cells in the presence of a culture medium, allowing said cells to grow and/or reproduce and isolating said cells.

In addition to the above, the present invention provides a method for the production of an organic compound comprising contacting anchorage-dependent eukaryotic cells or prokaryotic cells capable of producing said organic compound with the ceramic material according to the invention in the presence of a culture medium, allowing said cells to reproduce and isolating said cells.

Organic molecules include such compounds as vitamins, lipids, nucleotides, hormones, amino acids as well as more complex molecules such as nucleic acids, polypeptides including enzymes, peptide and proteo-hormones, other proteins and even complex glycoproteins.

Preferably, said organic compound is a polypeptide or protein. In a particularly preferred embodiment, said polypeptide or protein is selected from the group consisting of erythropoietin, Factor VIII, Factor IX, Factor VIIa, tissue plasminogen activator, thyrotrophin-alpha, follicle stimulating hormone, interferon-beta 1a, alpha 1-antitrypsin, dornase-alpha, antibodies and antibody fragments, platelet-derived growth factor, hirudin and hepatitis B surface antigen, insulin, human growth hormone, GM-CSF, interferon-alpha 2a, interferon-alpha 2b, interferon-beta 1b, interleukin-11, beta-glucocerebrosidase, TNF-alpha, antibodies, and derivatives thereof.

The invention further provides an article for culturing anchorage-dependent eukaryotic cells or prokaryotic cells comprising the ceramic material according to the invention.

Moreover the invention provides a use of the ceramic material of the present invention for the production of the above mentioned articles.

In one embodiment, the article is a support that provides a surface area for the culture of anchorage-dependent eukaryotic cells and prokaryotic cells. The support can have various shapes and sizes. The shape and size of the support are not particularly limited as long as the support provides access of culture medium to the cells being cultured. The support can be formed as a monolithic structure, sheet, disc, plate, pellet, sphere, and irregular particle. In addition, the surface of the support can be smooth or rough or partially smooth or rough.

In one embodiment, the article is capable of acting as a vessel or platform for the culture of anchorage-dependent eukaryotic cells or prokaryotic cells.

Densely sintered sheets and plates with porous, rough surfaces can be arranged in sandwich form and then result in large permeable surfaces for cell culture. The strength of sheets and thin surfaces comprised of multiphasic ceramics allow such technology.

The article can be a bioreactor, preferably a fixed-bed bioreactor, fluid-bed bioreactor, roller bioreactor, hollow fiber bioreactor or a part thereof.

For example, porous formed parts of the ceramic material of the invention can be advantageously employed as or in fixed-bed bioreactors and hollow fiber reactors, densely sintered formed parts can be advantageously employed in roller bioreactors.

The bioreactors made of the ceramic material of the invention are all well suited for the methods of production of anchorage-dependent eukaryotic cells and prokaryotic cells according to the invention as well as for the methods for the production of organic molecules, in particular proteins and polypeptides, according to the invention.

In addition, biocompatible implants and medical delivery systems or parts thereof can be produced from the ceramic material according to the invention. Such materials are tolerated by the surrounding tissues in vivo without or with minimal incompatibility reactions and the surrounding cells cover the surfaces and optionally penetrate into the porous structures.

For example, densely sintered formed parts can be used as microporous implants.

In addition, porous ceramic material of the present invention can be used as drug delivery systems or parts there of. After introduction of pharmaceuticals into the pores of the ceramic material of the invention, the particles can be implanted into organs and can served for the protracted release of said pharmaceuticals (medical delivery systems).

The following examples are meant to further illustrate the invention without limiting the invention to the examples.

### Example 1

1000 g of a suspension of zirconium oxide fully stabilized with calcium (Ca-FSZ, 17 mol% CaO, HiPer Ceramics GmbH, Eichstädt, Germany ) with an average particle size of 0.5µm to 0.8 µm and a solid portion of 80 wt% are mixed with 16 g of binder (Optapix AC 95, Zschimmer & Schwarz, Lahnstein Germany). The slip is then mixed with vigorous stirring with 24 g ammonium dihydrogenphosphate. Ammonia develops. Prefabricated cylindrical particles of open-pored polyurethane foam (BASF, Ludwigshafen, Germany), diameter 4 mm, height 4 mm, pore size 80 ppi) are soaked with the slip. The articles soaked with the slip are dried in air for two days and then sintered in crucibles of aluminum oxide at 1500°C for 1 hour. The resulting macroporous articles are suitable for filling fixed-bed bioreactors.

### Example 2

1000 g of a suspension of aluminum oxide with an average particle size of 1 µm (75 wt%, HiPer Ceramics GmbH, Eichstädt, Germany) are mixed with 6 wt% ammonium molybdate (Merck, Darmstadt, Germany), 50 wt% granulated graphite with an average particle size of 200µm (Timrex, Berlin, Germany), 20 wt% graphite with an average particle size of < 50 µm ( Merck, Darmstadt, Germany), 40 g binder (Optapix AC 95, Zschimmer & Schwarz, Lahnstein, Germany) with vigorous mixing and dried in plaster moulds until a total moisture content of 18%. The resulting paste is formed to thin walled disks or tubes in plaster moulds. Sintering at 1300°C leads to porous construction parts for roller bioreactors or hollow fiber bioreactors.

### Example 3

1000 g of a suspension of 40% zirconium oxide and 60% silicon oxide (oxide portion of 70 wt%, HiPer Ceramics GmbH, Eichstädt, Germany) are mixed with 4 wt% ammonium tungstenate (Sigma-Aldrich, Taufkirchen, Germany) and 200 g binder (Optapix AC 95, Zschimmer & Schwarz, Lahnstein, Germany) and poured to a 0.5 mm thick sheet (dimensions 100 x 100 mm). The sheet, dried with caution, is calcined at 1150°C; then coated on both sides with the same suspension as described above in this example but which contains 60 wt% graphite with an average particle size of 10 µm. After 10 hours of drying at 100°C, the sheet is sintered at 1600°C for 2 hours.

### Example 4

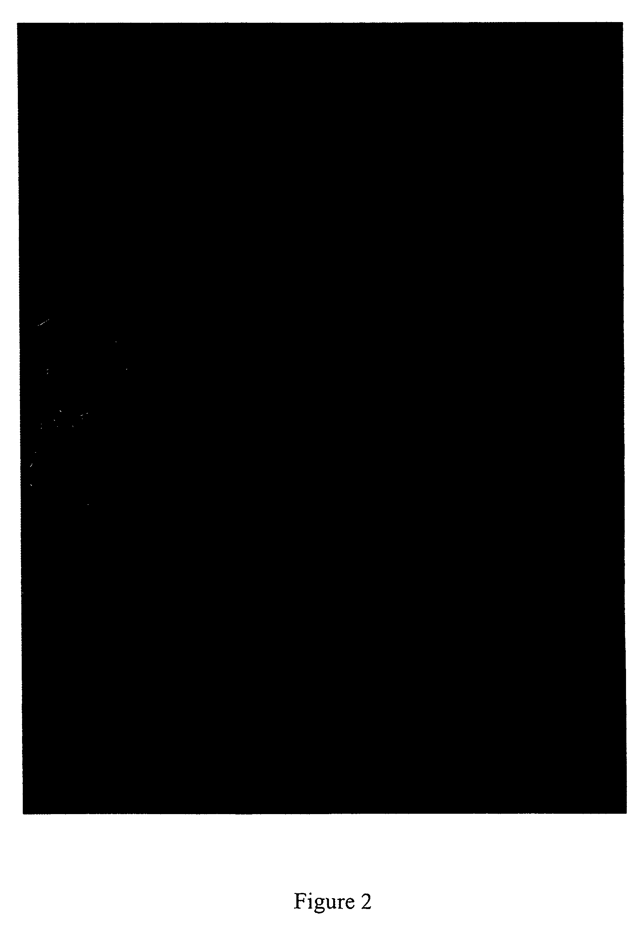

10² HeLa cells (DSMZ, Braunschweig, Germany) were suspended at room temperature in 1 ml RPMI 1640 medium containing 10 % CSF (Biochrom AG, Berlin). The cells were poured into a Petri dish (35 mm in diameter) filled with 2.5 ml RPMI 1640 medium. A biphasic, dense burned ceramic disc (zirconia with 2 % phosphate; 30 mm in diameter, 2 mm thick) was placed at the bottom of the Petri dish. The surface of the ceramic material was roughened by dragging. The cells were cultured at 37°C in an incubator with 5.5 % CO₂ atmosphere and adhered rapidly to the ceramic surface. After 24 h the number of cells was 5 x 10² (cell counting in a Becton Dickinson FACS). Cells were easy detachable by treatment with trypsin or other common methods. As can be seen from a Giemsa stained preparation in Fig. 2, cell growth was preferably along the micro-rough dragging lines of the ceramic material.

### Example 5

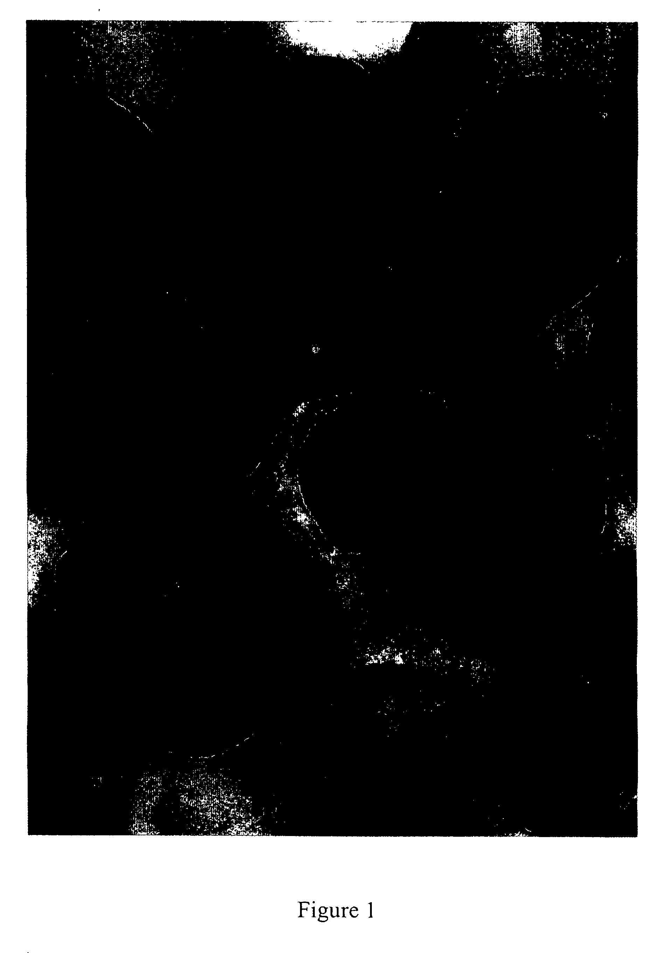

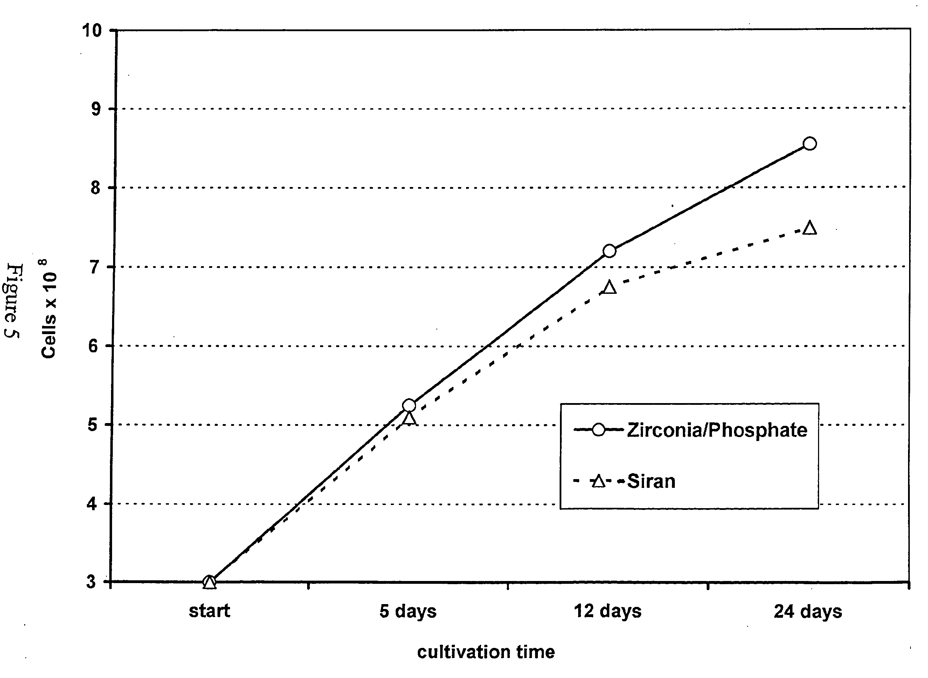

Macro-porous particles from zirconia and phosphate as described in example 1 were used as a solid bed in a 100 ml solid bed continuous flow bioreactor (Meridos KG, Bovenden, Germany). The surface of the solid bed was 1 m². A CHO-K1 cell clone bearing a stably introduced human albumin gene and grown in serum- and protein-free media was used for cultivation. The medium was Mix medium (Dulbecco's MEM and Ham's F12 (1:1)). Starting with 3 x 10⁸ seeding cells, 10¹¹ cells were grown after 8 weeks of cultivation. A total of 60 1 of medium was used. The cells were still fully viable at this time. The collected media contained a total of 100 mg recombinant human albumin estimated by immunoblotting. Fig. 1 shows the structure of the porous particles used in this trial. In Fig. 5, a comparison of the growth of the cells described above on the porous particles according to the invention is compared with that of the same solid bed volume from Siran particles.

As is demonstrated above, the growth of different cell types on the surface of Siran, a porous glass ceramic, is slower and the cell densities obtained were lower as compared to the multiphasic oxide ceramics of the invention.

While the invention has been described in detail and with reference to the examples thereof, it will be apparent to one skilled in the art that various changes and modifications can be made therein without departing from the spirit and scope thereof.

References cited
1. Looby D et al: The immobilisation of animal cells in fixed bed and fludized porous glass sphere reactors. In: de Bont JAM et al (eds), Elsevier Science Publishers B.V., Amsterdam 1990, 255-264
2. Amersham pharmacia biotech: Microcarrier cell culture. Uppsala, Sweden (1989)
3. Yamaji J and Fukuda H: Growth and death behaviour of anchorage-independent animal cells immobilized within porous support matrices. Applied Microbiol. Biotechnol. 37 (1992), 244-251
4. Wang G et al: High cell density perfusion culture of hybridoma cells for production of monoclonal antibodies in the celligen packed bed reactor. In: Kaminogawa S et al (eds): Animal cell technology 5 (1993) 463-469
5. Matsumara M et al: Bioreactor with a radial-flow nonwoven fabric bed for animal cell culture. In: Kaminogava S et al (eds): Animal cell technology 5 (1993), 433-442
6. Ray N G et al: Continuous cell cultures in fluidized-bed bioreactors. Cultivation of hybridomas and recombinant Chinese hamster ovary cells immobilized in collagen microspheres. Ann. N. Y. Sci. 589 (1990), 443-457
7. Pörtner r et al: Festbettreaktoren mit immobilisierten tierischen und menschlichen Zellen. Bioscope Sept. 1997, 25-33
8. Landgren B and Blümel G: Microcarriers in cell culture production. In: Subramanian G (ed.):Wyley-VCH, Weinheim, Vol. II (1999) 165-222
9. Mistler M: Schott Information 53/90, Mainz, Germany
10. Mitsuda S et al: Continuous production of tissue plasminogen activator by human embryonic lung diploid fibroblast imr-90 cells, using a ceramic bed reactor. Cytotechnology 6 (1990), 23-31
11. Alpha Bioverfahrenstechnik, Luckenwalde, Germany: Honey combs, alumina
12. Bauer G: Compatibility of oxide ceramic materials as components in biomedical engineering. Cerm. Forum Int. 70 (1993), 233-238
13. Toriyam M et al: High strength beta-tricalcium phosphate ceramics. V Estimation of biocompatibility by a tissue culture method. Nagoya Kogyo Gijutsu Shikensho Hokoko 39 (1990), 241-246
14. Nagase M et al: Immunglobulin G coating of ceramic powders enhances superoxide production. Nippon Seikeigeka Gakkai Zasshi 64 (1990), 593-601
15. Nagarajan V S and Rao K J: Structural, mechanical and biocompatibility studies of hydroxylapatite-derived composites toughened by zirconia addition. J. Mater. Chem. 3 (1993), 43-51
16. Lee et al : A study on the hydroxyapatite-zirconia composite bioceramics. Yoop Hakhoechi 29 (1991), 289-296 (Korean L.)
17. Qiu Q et al: Fabrication, characterisation and evaluation of bioceramic hollow microspheres used as microcarriers for 3-D bone tissue formation in rotating bioreactors. Biomaterials 20 (1999), 989-1001
18. Suchanek W et al: Hydroxiapatite ceramics with selected sintering additives. Biomaterials 18 (1997), 923-933
19. Li P et al: The role of hydrated silica, titania, and alumina in inducing apatite on implants. J. Biomed. Mater. Res. 28 (1994), 7-15
20. Ohgushi H et al: Al2O3 doped apatite-wollastonite containing glass ceramic provokes osteogenic differentiation of marrow stromal stem cells. J. Biomed.Mater. Res. 44 (1999), 381-388
21. Ogushi H et al: Al2O3 containing AW glass ceramic stimulates in vitro bone formation. Bioceram.,Proc. Int. Symp. Ceram. Med. 11 (1998), 261-264
22. Loty C et al: In vitro bone formation on a bone-like apatite layer prepared by a biomimetic process on a bioactive glass-ceramic. J. Biomed. Mater. Res. 49 (2000), 423-434
23. Suzuki T et al: The adhesivness and growth of anchorage-dependent animal cells on biocompatible ceramic culture carriers. J. Ferm. and Bioeng. 72 (1991), 450-456
24. US 5,976,454
25. US 5,171,720
26. US 6,013,591
27. US 5,990,381
28 US 5,232,878
29 WO 98/51981
30. EP 705609
31. EP 202107
32. EP 1038539
33. JP 2000102600
34. EP 705609
35. JP03131580
36. EP 241384
37. JP06006506
38. JP01036381
39. US 6113993
40. WO 99/26673
41. JP 07252112
42. DE 4113021

## Claims

1. Ceramic material comprising a crystalline core oxide ceramic dispersed with an amorphous polyanionic intergranular phase.

2. Ceramic material according to claim 1, wherein an oxide of the crystalline core oxide ceramic is preferably selected from the group consisting of zirconium (IV) oxide, aluminum oxide, silicon (IV) oxide, titanium (IV) oxide, hafnium (IV) oxide, cerium (IV) oxide and mixtures thereof.

3. Ceramic material according to claim 1 or 2, wherein a polyanionic compound in the amorphous intergranular phase is selected from the group consisting of phosphates, manganates, molybdates, tungstenates or mixtures thereof.

4. Ceramic material according to any one of claims 1 to 3, wherein a polyanionic compound is present in an amount of about 0.5% to about 30% with respect to the weight of the total ceramic material after sintering.

5. Ceramic material according to any one of claims 1 to 4, wherein a polyanionic compound is present in an amount of about 1% to about 10%.

6. Ceramic material according to any one of claims 1 to 5, wherein the surface area is about 1000 cm²/ml to about 6000 cm²/ml.

7. Ceramic material according to any one of claims 1 to 6, wherein the porosity is about 75% to about 90%.

8. Ceramic material according to any of claims 1 to 7 in the form of a particle, pellet, sphere, disc, plate, film or monolithic element.

9. Article for culturing anchorage-dependent eukaryotic cells or prokaryotic cells comprising the ceramic material according of claims 1 to 8.

10. An article according to claim 9, wherein the article is a bioreactor, fixed-bed bioreactors, fluid-bed bioreactors, roller bioreactors, hollow fiber bioreactors or a part thereof.

11. An article according to claim 9, wherein the article is a prosthetic device or implant.

12. Use of the ceramic material according to any of claims 1 to 8 as a bioreactor or part thereof.

13. Use of the ceramic material according to any of claims 1 to 8 as a or a prosthetic device, implant or drug delivery system or part thereof.

14. Method for the production of anchorage-dependent eukaryotic cells or prokaryotic cells comprising contacting the ceramic material according of claims 1 to 8 with eukaryotic cells or prokaryotic cells in the presence of a culture medium, allowing said cells to reproduce and isolating said cells.

15. Method according to claim 14, wherein the eukaryotic cells are naturally occurring or genetically altered human cells or cells derived therefrom.

16. Method for the production of an organic compound comprising contacting anchorage-dependent eukaryotic cells or prokaryotic cells capable of producing said organic compound with the ceramic material according of claims 1 to 8 in the presence of a culture medium, allowing said cells to reproduce and isolating said cells.

17. Method for the production of an organic compound according to claim 16, wherein said organic compound is a polypeptide or protein.

18. Method for the production of an organic compound according to claim 17, wherein said polypeptide or protein is selected from the group consisting of erythropoietin, Factor VIII, Factor IX, Factor VIIa, tissue plasminogen activator, alpha 1-antitrypsin, thyrotrophin-alpha, follicle stimulating hormone, interferon-beta 1a, dornase-alpha, antibodies and antibody fragments, platelet-derived growth factor, hirudin and hepatitis B surface antigen, insulin, human growth hormone, GM-CSF, interferon-alpha 2a, interferon-alpha 2b, interferon-beta 1b, interleukin-11, beta-glucocerebrosidase, TNF-alpha, antibodies, and derivatives thereof.

19. Method for the production of a ceramic material according to any of the previous claims comprising the steps of mixing a slip comprising:
a) one or more oxides capable of forming a crystalline oxide ceramic at a temperature of about 1150 to about 1600°C
b) one or more polyanionic compounds capable of forming an amorphous polyanionic intergranular phase at a temperature of about 1150 to about 1600°C
c) one or more binders, liquidizers and/or support materials that volatilize or burns off by a sintering temperature of about 1150°C to about 1600°C.
and sintering said slip, or a formed green body comprising said slip and a material that is capable of being burned off during sintering at a temperature range from about 1150°C to about 1600 °C.

20. Slip, pressing powder or plastic ceramic material comprising
a) one or more oxides capable of forming a crystalline oxide ceramic at a sintering temperature of about 1150°C to about 1600°C
b) one or more polyanionic compounds capable of forming an amorphous polyanionic intergranular phase at a sintering temperature of about 1150°C to about 1600°C and, optionally,
c) one or more binder(s), liquidizer(s) and/or support material(s) that volatilize or burns off by a sintering temperature of about 1150°C to about 1600°C.

21. Green form comprising a slip, pressing powder or plastic ceramic material according to claim 20.
